# EUROPEAN PATENT SPECIFICATION

(11) **EP 1 773 390 B1**
(45) Date of publication and mention of the grant of the patent: **29.04.2015**
(21) Application number: 05785042.2
(22) Date of filing: 19.05.2005
(51) Int. Cl.: A61P 31/00, C07K 16/40, A61K 39/395

(54) **IMPROVED THERAPEUTIC AGENT FOR INOS GENERATING ILLNESS**
VERBESSERTE BEHANDLUNGSMITTEL FÜR INOS-ERZEUGENDE ERKRANKUNGEN
AGENT THÉRAPEUTIQUE AMÉLIORÉ DESTINÉ À DES MALADIES GÉNÉRANT DU NOSI

(30) Priority: 19.05.2004 US 849768
(43) Date of publication of application: 18.04.2007
(73) Proprietor: Webber, Robert J., Las Vegas, NV 89135 (US); Webber, Douglas S., Benicia, CA 94510 (US); Dixon, Thelma H., Berkeley CA 94705 (US)
(72) Inventor: Webber, Robert J., Las Vegas, NV 89135 (US); Webber, Douglas S., Benicia, CA 94510 (US); Dixon, Thelma H., Berkeley CA 94705 (US)
(74) Representative: Modiano, Micaela Nadia
(86) International application number: PCT/US2005/017962
(87) International publication number: WO 2005/120569

(56) References cited:
- WO-A-03/012026
- US-B1- 6 531 578
- THOMPSON J ET AL: "Effect of iNOS inhibitors on LPS-induced exhaled nitric oxide in rats" NITRIC OXIDE, vol. 4, no. 3, 2000, pages 291-292, XP009016396 & FIRST INTERNATIONAL CONFERENCE ON BIOLOGY, CHEMISTRY, AND THERAPEUTIC APPLICATIONS OF NITRIC OXIDE; SAN FRANCISCO, CALIFORNIA, USA; JUNE 03-07, 2000 ISSN: 1089-8603

## Description

### BACKGROUND OF THE INVENTION

The present invention relates to a novel and useful therapeutic agent for the removal or neutralization of particulate iNOS in the blood, providing treatment for systemic inflammatory response syndrome (pre-sepsis), sepsis, severe sepsis, and septic shock.

Nitric oxide synthase (NOS) is an enzyme which is found in humans. Three isoforms of NOS have been identified. In the body nNOS and eNOS are constitutively expressed in the cells in which they are found. However, iNOS is not constitutively expressed, but is known to be induced by a number of cytokines, lipopolysaccarides (LPS), and other mediators of the inflammatory response. Specifically, iNOS has been associated as indicating certain pathological disease states. Notably, iNOS in the blood heralds the onset of sepsis, severe sepsis, and septic shock conditions in humans. Sepsis is estimated to kill more than 200,000 people annually in the United States alone. Of the persons who develop sepsis thirty percent die from this pathophysiology.

Reference is made to United States Patent 6,531,578 in which monoclonal antibodies are described that are specific for the recognition of iNOS in humans without cross-reacting with human eNOS or nNOS.

An immunoassay using such monoclonal antibody is capable of detecting the presence of sepsis within a very short period of time, a matter of minutes, when compared to the prior art tests which required several days to complete and obtain results. If sepsis is treated aggressively after recognition of its existence, persons afflicted with sepsis have a much better chance of surviving. Treatment of sepsis has been limited to known antibacterial, antifungal, and antiviral treatments. Such treatments have achieved limited success even with the rapid recognition of the presence of sepsis in a human.

An article entitled "Cloning and Characterization of Inducible Nitric Oxide Synthase from Mouse Macrophages", Xie et al, Science, 256: 225-228 (1992), reported the cloning and isolation of iNOS. The iNOS enzyme was described as a soluble cytoplasmic protein.

Subsequently, articles entitled "Nitric Oxide: Novel Biology with Clinical Relevance", Billiar, Ann Surg, 221#4: 339-349 (1995); "Nitric Oxide: Pathophysiological Mechanisms", Gross et al, Annu Rev Physiol, 57: 737-769 (1995); "The Cell Wall Components Peptidoglycan and Lipoteichoic Acid from Staphylococcus Aureus Act in Synergy to Cause Shock and Multiple Organ Failure", De Kimpe et al, Proc Natl Acad Sci USA, 92: 10359-10363 (1995); "Mechanism of Gram-Positive Shock: Identification of Peptidoglycan and Lipoteichoic Acid Moieties Essential in the Induction of Nitric Oxide Synthase, Shock, and Multiple Organ Failure", Kengatharan et al, J Exp Med, 188#2: 305-315 (1998); and "Induction of Nitric Oxide Synthase in RAW 264.7 Macrophages by Lipoteichoic Acid from Staphylococcus aureus: Involvement of Protein Kinase C- and Nuclear Factor-κB-Dependent Mechanisms", Kuo et al, J Biomed Sci, 10: 136-145 (2003), point to the fact that the lipopolysaccharide (LPS) cell-wall component of gram-negative bacteria, the lipoteichoic acid and peptidoglycan cell-wall components of gram-positive bacteria, fungi, and viruses can induce iNOS expression *in vivo* and *in vitro* in a wide variety of cell types.

Articles entitled "Mechanisms Of Suppression Of Macrophage Nitric Oxide Release By Transforming Growth Factor Beta", Vodovotz et al, J Exp Med, 178#2: 605-613 (1993); "Vesicle Membrane Association Of Nitric Oxide Synthase In Primary Mouse Macrophages", Vodovotz et al, J Immunol, 154#6: 2914-2925 (1995); and "Bladder Instillation And Intraperitoneal Injection Of Escherichia coli Lipopolysaccharide Up-Regulate Cytokines And iNOS In Rat Urinary Bladder", Olsson et al, J Pharmacol Exp Ther, 284#3: 1203-1208 (1998), have shown that since the discovery of iNOS in mouse macrophages, its intracellular location is not exclusively in the cytosol. In fact vesicle-associated iNOS has been recognized.

Articles entitled "Caveolin-1 Down-Regulates Inducible Nitric Oxide Synthase Via The Proteasome Pathway In Human Colon Carcinoma Cells", Felley-Bosco E et al, Proc Natl Acad Sci USA, 97#26: 14334-14339 (2000); "Macrophage Nitric Oxide Synthase Associates With Cortical Actin But Is Not Recruited To Phagosomes", Infect Immun, Webb JL et al, 69#10: 6391-6400 (2001); "Epithelial Inducible Nitric-Oxide Synthase Is An Apical EBP50-Binding Protein. That Directs Vectorial Nitric Oxide Output", Glynne PA et al, J Biol Chem, 277#36: 33132-33138 (2002); "Caveolin-1-Mediated Post-Transcriptional Regulation Of Inducible Nitric Oxide Synthase In Human Colon Carcinoma Cells", Felley-Bosco E, Biol Res, 35#2: 169-176 (2002); "Heat Shock Protein 90 As An Endogenous Protein Enhancer Of Inducible Nitric-Oxide Synthase", Yoshide M et al, J Biol Chem, 278#38: 36953-36958 (2003); "Protein Interactions With Nitric Oxide Synthase: Controlling The Right Time, The Right Place, And The Right Amount Of Nitric Oxide", Kone BC et al, Am J Physiol Renal Physiol, 285#2: F178-F190 (2003); and "Protein-Protein Interactions Involving Inducible Nitric Oxide Synthase", Zhang W et al, Acta Physiol Scand, 179#2: 137-142 (1997), have also reported that when induced cells are lysed and fractionated by centrifugation, iNOS is found in the particulate fraction.

Inducible NOS (iNOS) has also been found associated with a number of other proteins through a protein-protein interaction. Such protein-protein interactions (other proteins associated with iNOS) include cortical actin, EBP 50 (ezrinredixin-moesin-binding phosphoprotein 50), caviolin-1, Hsp90 (heat shock protein 90), kalirin, NAP110 (NOS-associated protein 1.10 kd), and Rac-GTPases. These protein-protein interactions have been found to localize iNOS to specific regions or structures within cells. Upon cell lysis and fractionation by centrifugation, either through vesicle association or by protein-protein interaction, a portion of the supposedly soluble iNOS protein has been shown to partition into the particulate fraction.

WO 03/012026 revealed the fact that iNOS found free in the liquid portion of the blood of a patient, i.e. plasma, indicates such patient has sepsis or will develop sepsis within the next 24 to 48 hours. Using a very sensitive chemiluminescent sandwich enzyme immunoassay (EIA), such plasma iNOS can be used as a very specific biochemical marker for the onset of sepsis. The heretofore referenced chemiluminescent sandwich (EIA) was based upon two of the anti-iNOS monoclonal antibodies (MAbs) of a panel of anti-iNOS antibodies which are disclosed in United States Patent No. 6,531,578, mentioned heretofore.

Although the detection of iNOS in the blood of patients is greatly aided in the treatment of those patients by conventional therapies, an improved therapy would be a notable advance in the medical field.

As further background art, Thompson J et al., "Effect of iNOS inhibitors on LPS-induced exhaled nitric oxide in rats", NITRIC OXIDE, vol. 4, no. 3, 2000, pages 291-292, XP009016396, & First International Conference on Biology, Chemistry, and Therapeutic Applications of nitric oxide; San Francisco, California, USA; June 03-07, 2000, ISSN: 1089-8603 discloses the use of iNOS inhibitors aminoguanidine and N-iminoethyl-lysine to treat asthma or other inflammatory conditions of the lung.

### BRIEF SUMMARY OF THE INVENTION

The present invention provides a therapeutic agent for the treatment of an illness selected from the group consisting of systemic inflammatory response syndrome, sepsis, severe sepsis, and septic shock, comprising a monoclonal antibody recognizing human iNOS.

In one embodiment of the therapeutic agent, the monoclonal antibody recognizing human iNOS comprises a monoclonal antibody neutralizing a cellular effect caused by a form of hiNOS, and the form of hiNOS is selected from the group consisting essentially of particulate hiNOS, fragments of particulate hiNOS, vesicle associated hiNOS, vesicle associated fragments of particulate hiNOS, particulate hiNOS associated with another protein, and fragments of particulate hiNOS associated with another protein. In this embodiment, the monoclonal antibody may be selected from the group consisting essentially of mouse anti-hiNOS monoclonal antibody, mouse-human chimeric anti-hiNOS monoclonal antibody, humanized anti-hiNOS monoclonal antibody, and human anti-hiNOS monoclonal antibody.

The present invention also provides a therapeutic device for the treatment of an illness in a mammalian subject generating hiNOS in its blood, in which the illness is selected from the group consisting of systemic inflammatory response syndrome, sepsis, severe sepsis, and septic shock comprising a device coated with a monoclonal antibody recognizing human iNOS.

In one embodiment of the device, the monoclonal antibody recognizing human iNOS comprises a monoclonal antibody neutralizing a cellular effect caused by a form of hiNOS, and the form of hiNOS is selected from the group consisting essentially of particulate hiNOS, fragments of particulate hiNOS, vesicle associated hiNOS, vesicle associated fragments of particulate hiNOS, particulate hiNOS associated with another protein, and fragments of particulate hiNOS associated with another protein.

In a further embodiment of the device, the monoclonal antibody is selected from the group consisting essentially of mouse anti-hiNOS monoclonal antibody, mouse-human chimeric anti-hiNOS monoclonal antibody, humanized anti-hiNOS monoclonal antibody, and human anti-hiNOS monoclonal antibody.

The therapeutic agent of the present invention may take the form of a monoclonal antibody recognizing human iNOS without cross-reacting with eNOS or nNOS. Such monoclonal antibody may constitute a mouse anti-hiNOS monoclonal antibody or mouse-human chimeric anti-hiNOS monoclonal antibody. In this regard, the iNOS recognized is believed to comprise the particulate fraction of the iNOS. Such monoclonal antibody may neutralize particulate iNOS which may be found in membrane-associated particulate iNOS, vesicle-associated particulate iNOS, or particulate iNOS in association with at least one other protein. It has been found that the illness most normally associated with the generation of iNOS in the blood of a patient is systemic inflammatory response syndrome (pre-sepsis), sepsis, severe sepsis, or septic shock. Moreover, the monoclonal antibody may be mouse anti-iNOS monoclonal antibody, mouse-human chimeric anti-iNOS monoclonal antibody, humanized anti-iNOS monoclonal antibody, or human anti-iNOS monoclonal antibody. Also, the therapeutic treatment disclosed herein is capable of removing iNOS from the blood of a mammalian subject by association with the monoclonal antibody. In such a case, means for achieving this result is also provided as disclosed above. Such means may take the form of a device coated with a monoclonal antibody which binds human iNOS without cross-reacting with eNOS or nNOS.

It may be apparent that a novel and useful therapeutic agent for the treatment of an illness in a mammalian subject generating iNOS has been hereinabove described.

It is therefore an object of the present invention to provide a therapeutic agent for the treatment of an illness in a subject generating iNOS which is safe and effective.

Another object of the present invention is to provide a therapeutic agent for the treatment of a malady in a mammalian subject generating iNOS which either neutralizes or removes iNOS from the blood of the patient.

Another object of the present invention is to provide a therapeutic agent for the treatment of a malady in a mammalian subject generating iNOS which is easily manufactured using known biochemical and immunological techniques.

Another object of the present invention is to provide a therapeutic agent for the treatment of an illness in a mammalian subject generating iNOS which neutralizes the cellular effects of iNOS in various forms.

Another object of the present invention is to provide a therapeutic agent for the treatment of an illness in a mammalian subject generating iNOS which inhibits cellular binding of iNOS in various forms.

Yet another object of the present invention is to provide a therapeutic agent for the treatment of an illness in a mammalian subject generating hiNOS which inhibits the cellular binding of forms of hiNOS.

A further object of the present invention is to provide a therapeutic agent for the treatment of an illness in a mammalian subject generating iNOS in the blood which is capable of saving lives.

The invention possesses other objects and advantages especially as concerns particular characteristics and features thereof which will become apparent as the specification continues.

### BRIEF DESCRIPTION OF THE SEVERAL VIEWS OF THE DRAWING

Fig. 1 is a photograph of a field of peripheral blood mononuclear cells (PBMCS) from a patient showing only one immunostaining positive cell (white), located at arrowhead, and a small iNOS containing vesicle (white), located at arrow, reacting with the indirect-fluorescent-labeled anti-iNOS monoclonal antibody 2A1-F8 in a field with numerous other non-reacting cells (very pale), at 200X.
Fig. 2 is a photograph of a field of PBMCS showing a large percentage of the cells containing iNOS and cellular associated iNOS containing vesicles (arrows) which are immunostained with the anti-iNOS monoclonal antibody 2A1-F8 by an indirect immunofluorescent assay (IFA) procedure, at 200X.
Fig. 3 is a photograph showing a less crowded field of PBMCS from that of Fig. 2 from a different septic patient than Fig. 2, in which iNOS-containing vesicle (presumably apoptotic bodies) are separate from the cells, at 200X.
Fig. 4 is a photograph having three panels, A, B, and C in which a common area is shown sequentially in UV light(A), phase-contrast light(B), and a combination of UV and phase-contrast light(C), at 200X, showing a large cluster of iNOS-containing vesicles (arrows).
Fig. 5 is a photograph of a PBMC from a patient photographed in UV light indicating a partially disrupted cytoplasmic membrane associated with iNOS-containing globules (presumably pre-apoptotic bodies), revealed by the IFA reaction with anti-iNOS monoclonal antibody 2A1-F8, at 400X.
Fig. 6 is a photograph of a PBMC photographed in UV light from the same patient as that shown in Fig. 5 indicating an iNOS immunopositive staining cell in the process of disintegration, and releasing iNOS-containing vesicles (presumably apoptotic bodies) at 200X.
Fig. 7 is a photograph of a western immunoblot showing the soluble and particulate fractions of iNOS where, lane 1 is molecular weight standards, lane 2 is the induced soluble fraction at 5µl, lane 3 is the induced soluble fraction at 2.5µl, lane 4 is the induced particulate fraction at 5µl, lane 5 is the induced particulate fraction at 2.5µl, lane 6 is an iNOS standard, and lane 7 is the molecular weight standards.
Fig. 8 is a chart illustrating the 48 hour survival of mice primed with LPS and four hours later administered the chemical entities described in Example 1.
Fig. 9 is a western immunoblot following SDS-PAGE indicating the removal of iNOS from the particulate fraction described in Example 2, in which lane 1 is the molecular weight standards, lane 2 is an iNOS standard at 5µl, lane 3 is the induced particulate fraction at 5µl, lane 4 is the induced particulate fraction at 2.5µl, lane 5 is the anti-iNOS MAb coated MAG-BEAD depleted particulate fraction at 5µl, lane 6 is the anti-iNOS.MAb coated MAG-BEAD depleted particulate fraction at 2.5µl, lane 7 is an iNOS standard at 5µl, and lane 8 is the molecular weight standards.
Fig. 10 is a photograph of the immunostaining of iNOS bound to anti-iNOS monoclonal antibodies attached to the MAG-BEADs used in Example 2.
Fig. 11 is a chart illustrating the seven day survival of mice primed with LPS and four hours later administered certain chemical entities, described in Example 2.
Fig. 12 is a chart illustrating the seven day survival of mice primed with LPS and four hours later administered certain chemical entities, described in Example 3.
Fig. 13 is a chart illustrating the seven day survival of mice primed with LPS and four hours later administered certain chemical entities, described in Example 4.
Fig. 14 is a graph depicting the colormetric titration on a microtiter plate sensitized with peptide F6 of mouse anti-hiNOS MAb 24H9-1F3 (MOUSE MAb I) using goat anti-mouse IgG-HRP conjugate as detection antibody, of humanized anti-hiNOS MAb 24H9-1F3 (Humanized MAb I-a) using goat anti-human IgG-HRP conjugate as detection antibody, or of humanized anti-hiNOS MAb 24H9-1F3 (Humanized MAb I-b) using goat anti-human IgG-HRP conjugate as detection antibody.
Fig. 15 is a graph showing an ELISA titration chemiluminescent sandwich immunoassay titration of humanized anti-hiNOS MAbs 1E8-B8 (MAbA), 2D10-2H9 (MAbD), or 24H9-1F3 (MAbI) each with a "capture" anti-hiNOS antibody, each with 10 fmoles of hiNOS, and each with anti-human IgG-HRP conjugate as a "detection" antibody.
Fig. 16 is a bar graph indicating the survival rate of LPS-primed mice in controlled experiments employing humanized MAbs 1E8-B8 and 24H9-1F3, with P values calculated by Student's T-test.
Fig. 17 is a Western blot confirming the presence of proteins found in association with hiNOS or fragments of hiNOS in the particulate fraction.
Fig. 18 is a pair of digital image panels, where panel "A" shows extra cellular vesicles resulting from hypotonic shock of cytokine induced DLD-1-5B2 cells displaying intense immunofluorescent staining for iNOS, and panel "B" shows vesicles fluorescently immunostained for hiNOS surrounded by hypotonicaly lysed cells.
Fig. 19 is a Western blot that shows molecular weight standards (lane 1); intact hiNOS contained in the high speed supernatant (lanes 2 and 3); intact hiNOS and two fragments of hiNOS contained in the low speed particulate fraction (lanes 4 and 5); and no hiNOS contained in the high speed particulate fraction.

For a better understanding of the invention reference is made to the following detailed description of the preferred embodiments thereof which should be taken in conjunction with the prior described drawings.

### DETAILED DESCRIPTION OF THE PREFERRED EMBODIMENTS OF THE INVENTION

Various aspects of the present invention will evolve from the following detailed description of the preferred embodiments thereof and examples which should be taken together with the hereinbefore described drawings.

In clinical trials, more than 340 human subjects were enrolled, and over 1,200 blood samples were collected and analyzed to determine if the chemilumenescent EIA for iNOS, described in United States Patent 6,531,578 and WO 03/012026 prognosticate the onset of sepsis and monitored the course of the pathology. It was found that free iNOS (soluble iNOS) was present in the blood samples. Also, particulate iNOS, in the form of membrane associated particulate iNOS, vesicle associated particulate iNOS, or particulate iNOS in association with another protein (by protein-protein interaction), was present in some of the blood samples. Such particulate iNOS was not attached to any of the cells.

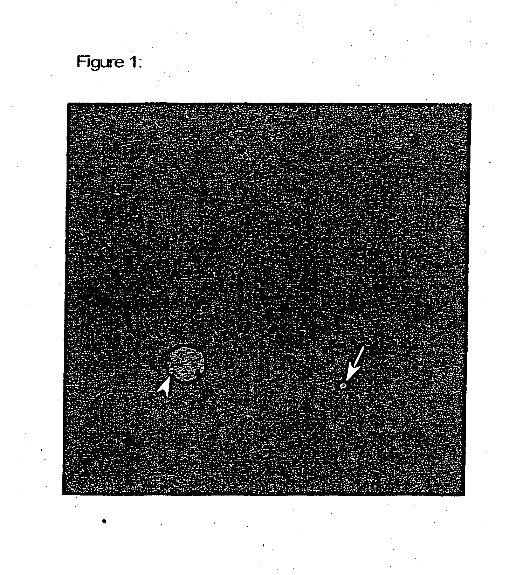

Figs. 1-6 showed the presence of human iNOS in peripheral blood mononuclear cells (PBMCs) and in vesicles/globules that are not cell associated. For example, Fig. 1 shows a PBMC cell that contains human iNOS. The iNOS-containing PBMC cell is present in a background of non-staining cells. It may be observed that the immunostaining in this PBMC cell is not evenly distributed, as might be expected by the typical distribution of cytoplasmic protein in a normal cell. The immunostaining material appears globular and is located at the peripheral rim of the cytoplasm (arrow head). The nuclear region does not contain iNOS and appears pale as a result of the thin layers of cytoplasm above and below the nucleus in the cell. Also, a single vesicle appears, that is not cell associated, which also contains human iNOS (full arrow). It is believed that the single vesicle which is intensely fluorescing may be an apoptotic body.

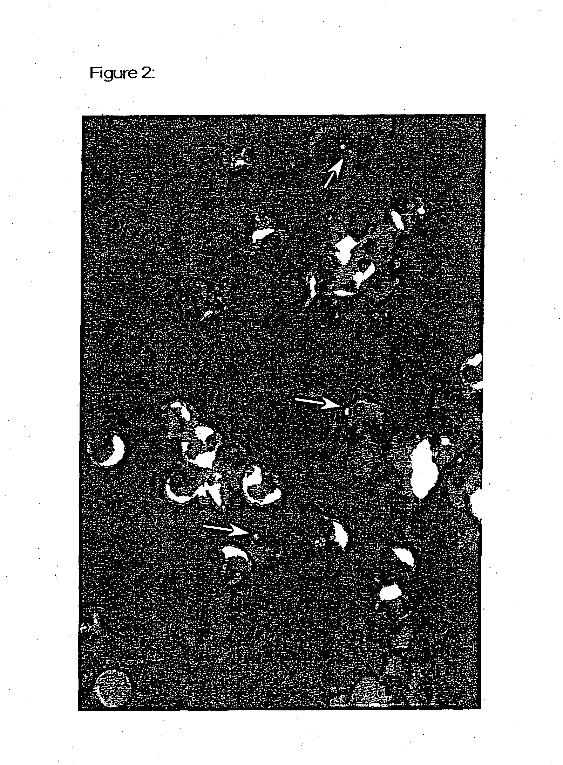

Turning to Fig. 2, many cells are shown each of which are immunostained for human iNOS. Also visible are numerous small extra cellular vesicles (apoptotic bodies), that are immunostained for human iNOS, too. The presumptively apoptotic bodies are present as small white dots located at the edges of the cells (full arrows). The nucleus of each cell is present in the non-fluorescently staining portions (dark region) of the cells. Also, other non-reacting cells are present in the background.

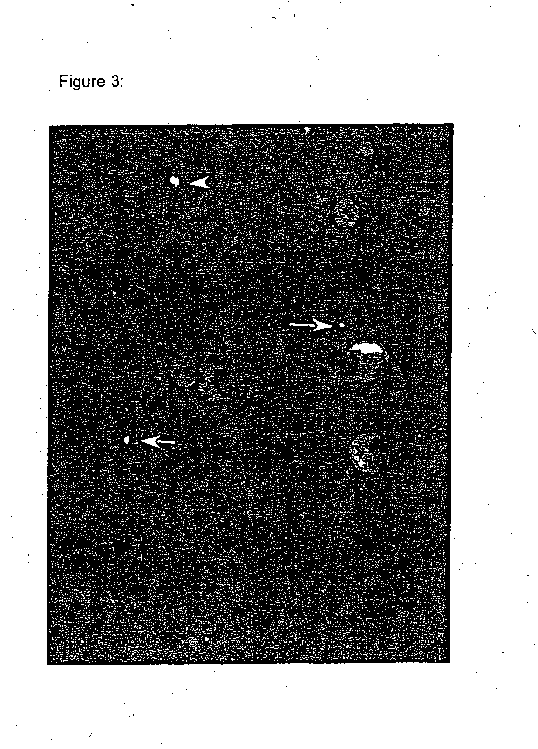

Fig. 3 depicts a relatively open field of PBMCS. Small extra cellular vesicles, presumably apoptotic bodies, appear as white dots and are separate from the cells (arrows). The immunostaining for iNOS with the anti-iNOS monoclonal antibody 2A1-F8 appears granular in these cells. The anti-iNOS monoclonal antibody 2A1-F8 is disclosed in U.S. Patent 6,531,578.

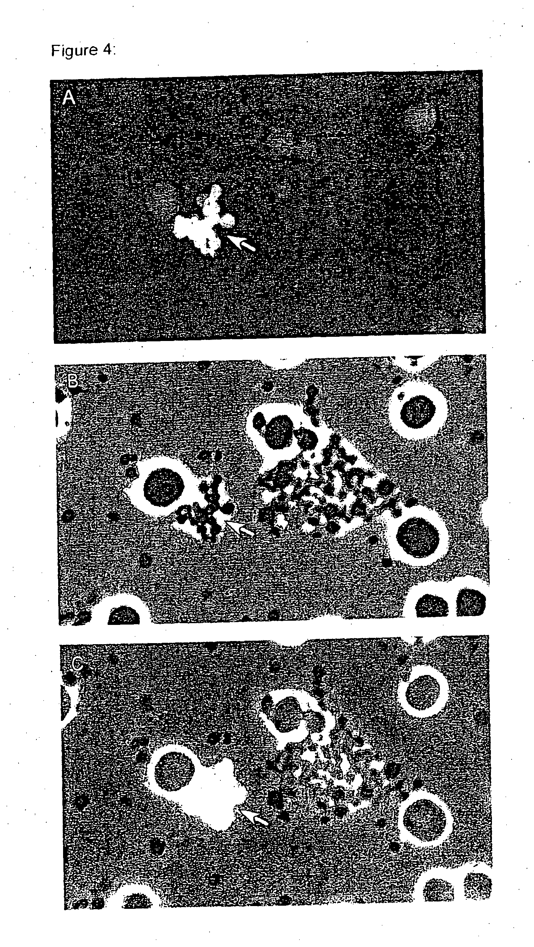

Fig. 4 depicts three photographs, in panels A, B, and C, with a common cluster of globules and cells. Panel A was photographed in UV light and reveals the immunostaining of globules located as a cluster. Some of the cells appear small, are shrunken, and are negative for iNOS immunostaining by IFA. An arrow appears next to the immunostained globules in a cluster.

Panel B, Fig. 4, was photographed in phase-contrast light, and again reveals the presence of the numerous extra cellular globules in the sample (arrow). The intact cells are shown as dark black bodies with a white halo that the results from the phase-contrast optics.

Finally, in panel C of Fig. 4, the same area as shown in panels A and B was photographed with a combination of UV light and phase-contrast optics. The white cluster of globules (arrow) indicate immunostaining with anti-iNOS monoclonal antibody 2A1-F8, demonstrating that the extra-cellular globules contain iNOS.

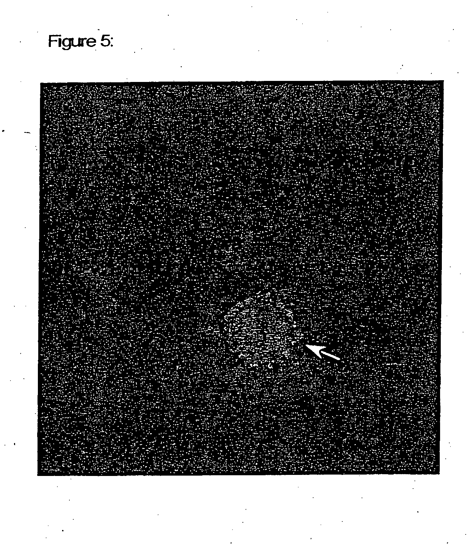

With reference to Fig. 5, a single PBMC, immunostaining for human iNOS, is in the process of "blebbing". In other words, the PBMC cell has a partially disrupted cytoplasmic membrane associated with iNOS containing globules. The pre-apoptotic bodies or apoptotic "blebs" are immunostained for human iNOS.

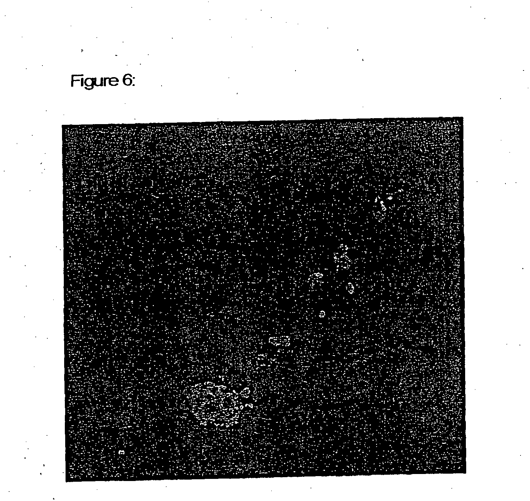

Fig. 6 depicts a single PBMC cell in the process of disintegration and releasing materials and vesicles that immunostained for human iNOS. The cell membrane has been disrupted and the iNOS-containing globules/vesicles are scattered.

Figs. 1-6 represent evidence for the existence of apoptotic bodies *in vivo.* All of the particulate or vesicle-associated iNOS was only found in samples from patients afflicted with sepsis, severe sepsis, or septic shock. The presence of apoptotic bodies as revealed in Figs. 1-6 in the blood stream of a human may be an indication of the presence of sepsis or an indication of the severity of the pathology of the same.

Since soluble iNOS and the particulate or vesicle-associated iNOS are only found in the blood of critically ill patients, the contribution to the pathology of systemic inflammatory response syndrome, sepsis, severe sepsis, or septic shock by these forms of iNOS was investigated. In other words, the presence of soluble or particulate iNOS in the circulation was theorized to be deleterious to patients with sepsis or pre-sepsis. Consequently, it may be reasoned that removal or neutralization of iNOS from the blood stream of patients with sepsis or pre-sepsis conditions may constitute a possible therapeutic treatment for such illnesses.

In order to gather data that might confirm such hypothesis, a mouse model of sepsis was used in a series of experiments. Such tests were employed to determine whether or not soluble or particulate iNOS contributed to the pathology of systemic inflammatory response syndrome sepsis, severe sepsis, or septic shock. In addition, it was determined if removal or the neutralization of soluble or particulate iNOS helps diminish the sepsis pathology..

As heretofore stated, DLD-1-5B2 cells can be induced to produce human iNOS by the addition of a mixture of cytocytokines.

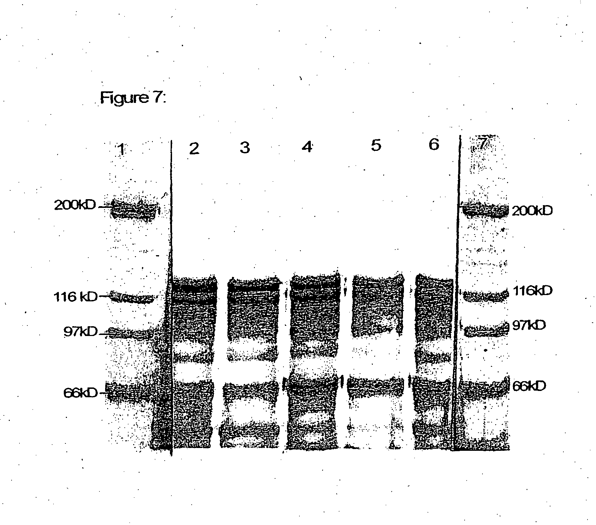

In an article entitled "Transcriptional Regulation of Human Inducible Nitric Oxide Synthase Gene In An Intestinal Epithelial Cell Line", Linn et al, Am J Physiol, 272: G1499-G1508 (1997), it was shown that DLD-1 cells can be induced to produce human iNOS. Also, when the induced cells are lysed, two types of iNOS can be isolated by centrifugation, a soluble iNOS fraction and a particulate iNOS fraction. Fig. 7 shows a Western immunoblot which indicates that the pooled soluble fraction of induced DLD-1-5B2 (a clone of DLD-1) cells contains iNOS (lanes 2 and 3) at the predicted molecular weight of 131 kD. Also, the particulate fraction of induced DLD-1-5B2 cells likewise contains iNOS (lanes 4 and 5) as shown by the band at 131 kD. Lane 6 contains an iNOS standard and lanes 1 and 7 contain standard proteins used as molecular weight markers at the indicated weights. In order to produce and isolate soluble and particulate fractions of iNOS from induced DLD-1-5B2 cell cultures, the following steps were followed:
1. DLD-1-5B2 cells were grown in culture starting from frozen cryo-preserved cells;
2. The expression of iNOS was induced in the cells;
3. The induced cells were harvested; and
4. The iNOS in the induced cells was isolated and fractionated into soluble and particulate fractions.

To grow and expand DLD-1-5B2 cells, a vile of cryo-preserved cells was obtained and thawed. The percent viability was calculated -it should be greater than 75 percent- by trypan blue exclusion prior to culturing the cells. Cells were transferred into a T-75 flask containing DLD-1-5B2 medium (90 percent DMEM and 10 percent FBS supplemented with PEN/Strept). Cells were incubated in a humidified atmosphere of 5 percent CO₂ in air at 37°C. The medium was changed every other day until the cells were almost confluent. Following such procedure, the medium was changed daily until the cell cultures were either split or induced. When the DLD-1-5B2 cells were near confluence in log-phase growth, the cells were split 1:6 to 1:10 into additional T-75 flasks.

DLD-1-5B2 cells were induced to express human iNOS using a mixture of rhIFNγ at 8.33 ng/ml, rhTNFα at 3.3ng/ml, and rhIL-1β at 3.3ng/ml for 18 hours. During the induction of iNOS, an increase in the quantity of the end products of NO production, nitrite and nitrate, was monitored to determine if iNOS was being produced by the induced cells. The Griess reaction was used to assay the quantity of nitrite contained in the culture medium, and the amount of nitrate after the enzymatic conversion of the nitrate to nitrite by the enzyme nitrate reductase.

The DLD-1-5B2 cells were harvested, 18 hours post-induction. To maximize induced cell recovery, all culture fluid from the induced flasks were transferred and combined into 50 ml sterile centrifuge tubes to collect "floater" cells. Each tube was centrifuged, and the spent medium was discarded. The "floater" cell pellet was set aside until ready to wash with PBS. All the T-75 flasks were washed with PBS to remove spent medium and its serum components. A mixture of trypsin/EDTA was incubated with the cells for five to ten minutes at 37°C to dislodge the cells from the surface of each flask. After the cells were freed from the plastic surface, heat-inactivated FBS was added to each flask to stop the trypsin reaction. The cells were then transferred to a screw capped centrifuge tube and collected by centrifugation. "Floater" cells were then combined with the trypsinized cell pellet. The pooled induced cells were washed three times with sterile PBS and collected by centrifugation after each wash. The washed cells were transferred into sterile tubes in a small volume of sterile PBS, and stored at -20°C until ready to process for the isolation and fractionation of iNOS.

The iNOS produced by the induced DLD-1-5B2 cells was then fractionated into soluble and a particulate fractions. The induced DLD-1-5B2 cells, which were previously harvested and frozen, were thawed in an ice water bath until the entire contents of the tube had melted. Occasional vortexing during the thawing aided the process. The cells were lysed by two rapid freeze/thaw cycles using dry ice. The lysed cells were centrifuged at 16,000 xg at 4°C for 30 minutes to pellet the particulate fraction. The supernatant containing the soluble iNOS was transferred and stored on ice. The pellets were resuspended in a small volume of ice cold sterile PBS, vortex mixed vigorously, and centrifuged at 16,000 xg at 4°C tor 30 minutes to pellet the particulate fraction. The resulting supernatant was pooled with the first supernatant. Such supernatant solution contained the isolated iNOS soluble fraction. The particulate fraction of iNOS was then stored at-20°C or used. The iNOS soluble fraction required stabilization by the addition to a final concentration of 2 percent normal horse serum, followed by storage in a frozen condition at -20°C.

In general, once the cryo-preserved cells are restarted by placing in culture, they reach log phase growth after a few days at 37°C in a humidified 5 percent CO₂/95 percent air atmosphere. Once the DLD-1-5B2 cells are in log-phase growth, a daily monitoring and feeding cycle is required for maximum iNOS expression, the DLD-1-5B2 cells should be induced with a mixture of three cytokines (IFNγ, TNFα, and IL-1β) two days past confluence and harvested 18 hours after the start of the induction. From starting up the cell culture to finishing the first harvest took approximately 11 days, inductions and harvests were a weekly routine thereafter. DLD-1 cells are available from ATCC (CAT.#CCL221). The DLD-1-5B2 cell line was derived by subcloning the DLD-1 cells using standard cloning techniques.

The effects of the soluble iNOS fraction and of the particulate iNOS fraction were tested on mice primed with a sub-lethal dose of LPS as a model of sepsis. This was done to determine the effects the two different fractions of iNOS protein had on the viability of the mice. The results of these experiments led to the discovery that the iNOS protein has a function in signaling death in the mice. Several experiments were conducted, and it was discovered that the membrane associated iNOS, rather than soluble fraction of iNOS, plays a role in causing death in this mouse model of sepsis. It was also discovered that antibodies found in United States Patent 6,531,578 protected mice from death caused by a sub-lethal dose of lipopolysaccharide (LPS) and particulate iNOS.

It is also found from the experiments that LPS priming of mice was necessary for the effect of the particulate iNOS to be exerted. Also, tne administration of the particulate iNOS fraction, without soluble iNOS, to LPS primed mice caused almost immediate death. Particulate iNOS by itself or in association with one or more proteins is believed to be responsible for the lethal effect observed in LPS primed mice. Removal of the iNOS in particulate form or in association with one or more proteins by absorption from solution stopped the lethal effects exerted by the administration of the particulate iNOS to the LPS primed mice. It was also found that different anti-iNOS MAbs varied in their individual ability to neutralize the lethality in the mice of particulate iNOS, in the form of particulate membrane associated iNOS, particulate vesicle associated iNOS or particulate iNOS in association with at least another protein. Also, lowering the dose of LPS and particulate iNOS increased the survival rate of the mice. However, the administration of anti-iNOS MAbs to LPS primed mice increased the seven day survival rate.

Further, humanized or chimeric anti-hiNOS monoclonal antibodies of the panel found in United States Patent 6,531,578 were developed utilizing genetic engineering procedures. Specifically, seven chimeric mouse/human anti-hiNOS monoclonal antibodies (MAbs) were generated by replacing the constant regions of mouse anti-hiNOS MAbs 1E8-B8, 2D10-2H9, 6A12-A12, 21C10-1D10, 21H11-2D2, 24B10-2C7, and 24H9-1F3, with human IgG₁ and Igκ constant regions. Total RNA was isolated from each of the seven hybridoma cell lines listed above. The RNA encoding immunoglobulin heavy chain (HC) and light chain (LC) was amplified by RT-PCR. The resulting PCR products were subcloned into a suitable cloning vector and sequenced. The prototype nucleotide sequence of each antibody chain was established from multiple cDNA clones. The precise location of immunoglobulin variable regions was determined by comparing the prototype sequences with the IMGT database (http://imgt.cines.fr). For each of the seven MAbs, one prototype HC-variable region clone and one prototype LC-variable region clone were selected for constructing the chimeric antibody. A second PCR was used to amplify the variable regions with the appropriate restriction sites added so that (1) the mouse HC-variable region could be inserted in frame into an expression vector already containing the human IgG₁ constant region, and (2) the mouse LC-variable region could be inserted into an expression vector already containing the human Igκ constant region. The DNA sequence of both variable regions was verified again to make certain the insertion occurred in frame as intended and no other mutations were introduced during the second PCR. Approximately 500 µg of each plasmid DNA was prepared.

For each antibody, the vector DNA encoding the.chimeric HC and the *dhfr* selection marker, and the vector encoding the chimeric LC and the neo selection marker were co-transfected into the *dhfr⁻* DUXB11 CHO cell line to produce the complete antibody molecule. A selection of stably transfected cells expressing both heavy and light antibody chains was carried out by culturing in nucleoside-free medium containing the neomycin analog, G418. Since the DUXB11 CHO *(dhfr⁻)* cells lacked the dehydrofolate reductase (DHFR) enzyme necessary for the synthesis of DNA, these cells also lacked the ability to grow in nucleoside-free medium. Introduction of the *dhfr* gene by co-transfection with two vectors, one of which contained a functional *dhfr* gene, restored these cells ability to grow in culture medium deficient in nucleosides.

In order to obtain sufficient expression of the recombinant proteins in mammalian systems, gene amplification was accomplished. This took place by subjecting the cells to growth in the presence of a selective pressure by adding increasing concentrations of methotrexate (MTX) to the culture medium. Since the MTX inhibited activity of the DHFR enzyme, the only cells to survive achieved a sufficient level of *dhfr* gene amplification, allowing them to overcome the inhibitory effect of the MTX. Since gene amplification extended to regions flanking the *dhfr* gene, the DNA encoding both chains of antibody also co-amplified during gene duplication.

Cells expressing three of the anti-hiNOS chimeric MAb DNA constructs generated (humanized 1E8-B8, humanized 2D10-2H9 and numanized 24H9-1F3) were amplified. Amplified cell pools expressing and secreting the highest levels of humanized MAb were chosen for collection of conditioned media containing the secreted humanized anti-hiNOS MAb. These media were used as starting material for the isolation of milligram quantities of the humanized anti-hiNOS MAbs, required for experiments to determine their binding properties.

Experiments were conducted to demonstrate that the genetically engineered chimeric mouse/human anti-hiNOS MAbs possessed the same binding properties as their respective cognate parent mouse anti-hiNOS MAbs. Tests showed that the anti-hiNOS antibody binding activity had been incorporated into a human IgG₁ molecule, and that few, if any, mouse IgG epitopes remained on the humanized MAbs. Further, these chimeric anti-hiNOS MAbs were used in experiments to demonstrate their usefulness (1) in removing particulate hiNOS, vesicle associated hiNOS, and particulate hiNOS associated with other proteins from solution as previously described for the parent mouse MAbs, and (2) in neutralizing the killing activity of particulate hiNOS in a *in vivo* mouse model of sepsis.

In addition, proteins associated with hiNOS in the particulate fraction of cytokine induced DLD-1-5B2 cells were found. Anti-hiNOS MAbs were immobilized on MAGBEADS to bind hiNOS associated with such proteins in the particulate fraction. After isolation the proteins were identified by recognition of their amino acid sequences through LC/MS/MS. Comparison to known protein sequences in the Gene Bank database produced reliable identifications.

Moreover immunofluorescent staining experiments were performed on the low speed pellet of cells lysed by hypertonic shock. Only the low speed pellet exhibited lethal effects on LPS primed mice, when compared to the high speed pellet and high speed supernatant. The anti-hiNOS MAbs of United States Patent 6,531,578 produced intense fluorescent staining in small vesicles or apoptotic bodies, similar to the vesicles observed in the blood of human septic patients, Figs. 1-6.

It was determined that the lethal activity contained in the particulate fraction of cytokine induced and lysed DLD-1-5B2 cells can be monitored specifically by use of an *in vivo* mouse model of sepsis. The lethal activity can be selectively removed from the particulate fraction using humanized anti-hiNOS MAb 1E8-B8 immobilized on beads, and when tested in such *in vivo* mouse model of sepsis, 100% of the animals survived that otherwise would have died had the particulate fraction not been treated with humanized anti-hiNOS MAb 1E8-B8 immobilized on a solid support. The lethal activity entity can be recovered from the immobilized. anti-hiNOS MAb by competing it off with synthetic peptide PS-5183 to which the anti-hiNOS MAb loaded on the bead has been previously shown to bind. When tested in.the *in vivo* mouse model of sepsis, the recovered material is lethal to LPS-primed mice.

The following examples are provided to further illustrate the present invention but are not deemed to limit the invention in any manner.

### EXAMPLE I

The two fractions of human iNOS, illustrated in Fig. 7, were investigated as to their effect on LPS primed mice as an animal model of sepsis. Prior to starting the experiment, soluble iNOS was removed from the soluble fraction by selective absorption onto MAG-BEADS coated with one or more of the anti-iNOS MAbs found in the United States Patent 6,531,578. Briefly, MAG-BEADS covalently bonded to goat anti-mouse IgG IgG were purchased from the Pierce Chemical Co. in Rockford, Illinois. Culture supernatant containing secreted anti-iNOS MAbs from clones 21C10-1D10, 2A1-F8, 1E8-B8 and 2D2-B2 were applied individually to aliquots of the suspended MAG-BEADS in order to load the MAG-BEADS with monoclonal antibodies specific for iNOS. The soluble fraction containing iNOS was diluted 1:2 and applied to pooled, washed, a resuspended anti-iNOS coated MAG-BEADS. The suspension was incubated overnight with gentle mixing to allow the soluble iNOS to bind to the anti-iNOS MAbs coated onto the MAG-BEADS before the tube containing the suspension was placed onto a magnetic rack. All the beads congregated on the sides of the tube next to the magnets. The resulting iNOS-depleted soluble fraction was transferred and diluted to a final volume to yield a 1:5 dilution as compared to the stock soluble fraction. A 1:5 dilution of the stock iNOS soluble fraction.was also prepared in sterile saline. Samples of the 1:5 iNOS-depleted soluble.fraction of the 1:5 diluted stock soluble fraction, and of the iNOS coated MAG-BEADS used to create the iNOS-depleted soluble fraction were all analyzed to determine if the soluble iNOS had been removed and to demonstrate that the soluble iNOS was bound to the anti-iNOS MAbs attached to the MAG-BEADS. These analyses showed that more than 90 percent of the soluble iNOS had been removed from the soluble fraction (iNOS-depleted soluble fraction), and that the iNOS was bound to the MAG-BEADS which had been loaded with the anti-iNOS MAbs.

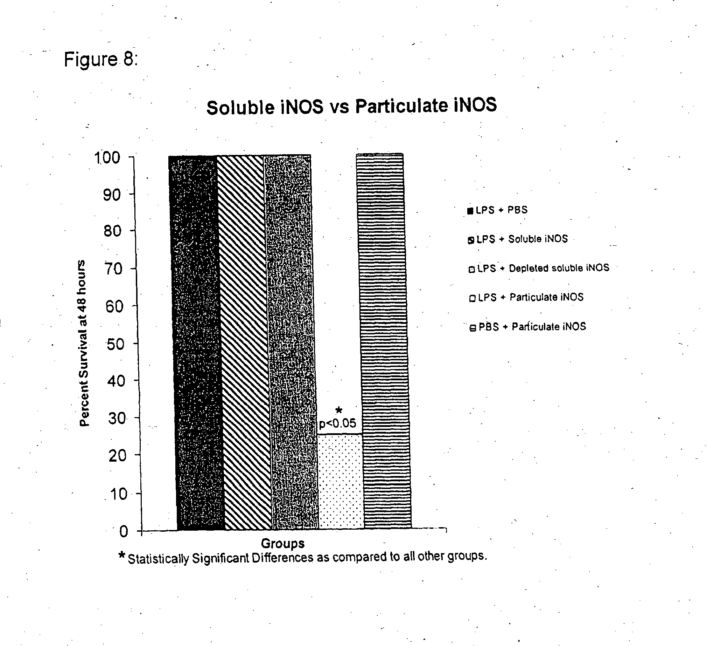

Groups of mice containing both genders were injected IP with sterile saline only, or with a sub-lethal dose of LPS (2mg/kg body weight of LPS Serotype 0111:B4 from E. coli, obtained from Sigma Chemical Co., Saint Louis, MO) in sterile saline. After four hours, only the mice injected with LPS became lethargic and developed diarrhea. The saline or LPS-primed mice were then given an additional tail vein injection of either saline or one of the following: the soluble fraction containing iNOS (soluble iNOS), the soluble fraction depleted of iNOS (iNOS-depleted soluble fraction), or a suspension of particulate iNOS produced by and isolated from induced DLD-1-5B2 cells. Fig. 8 shows the results of this experiment. None of the saline-primed mice showed any effect with any of the test samples. No effect was seen with the LPS-primed mice upon administration by tail vein injection of either a dose of saline, a dose of soluble iNOS, or a dose of iNOS-depleted soluble fraction. However, when the particulate fraction of iNOS was administered to the LPS prime mice, all the mice died almost immediately. No effect was seen on the saline-primed mice given the same dose of particulate iNOS. It was concluded that (1) LPS priming of mice was necessary for the effect of the particulate iNOS to be exerted, and (2) particulate iNOS, not soluble iNOS, when administered to LPS-primed mice caused an almost immediate death.

### EXAMPLE II

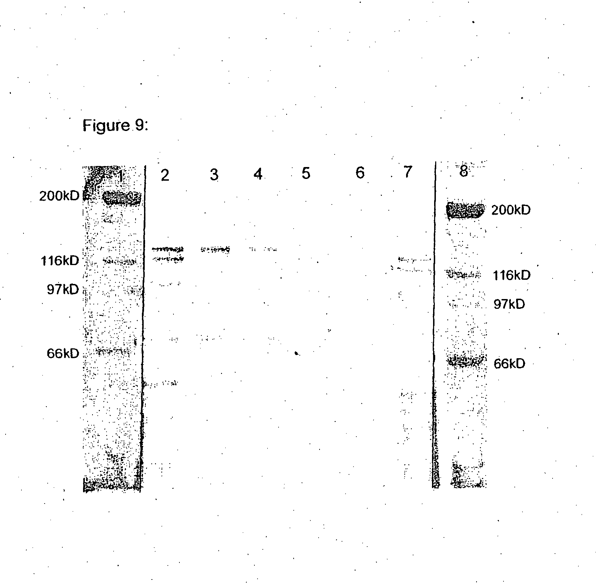

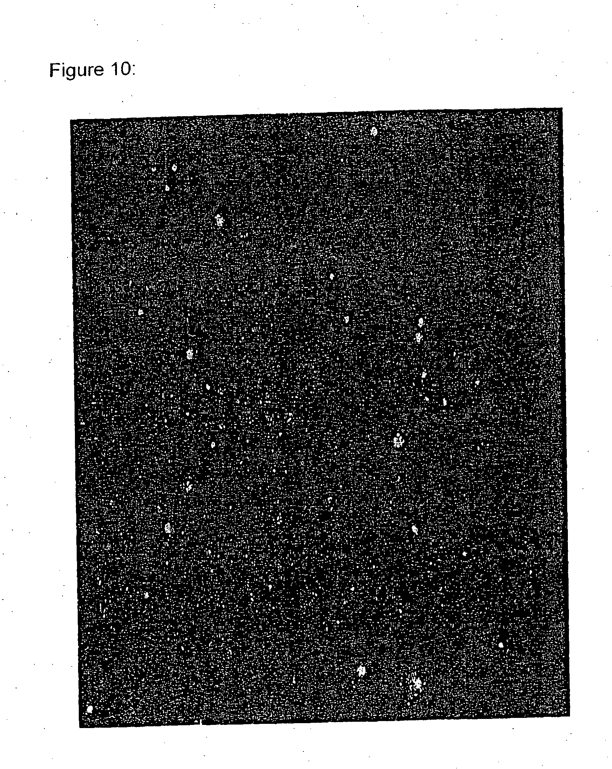

The anti-human iNOS MAbs found in United States Patent 6,531,578 were employed in order to investigate the inhibition of the killing effect seen with the particulate human iNOS in LPS-primed mice. Particulate iNOS was removed from the particulate fraction by selective absorption onto MAG-BEADS coated with the anti-iNOS MAbs as described in Example I. Fig. 9 represents the Western immuno blot confirming the selective removal of particulate iNOS from the particulate fraction. A similar procedure to the one described with respect to depletion of the soluble fraction in Example I was employed. Briefly, MAG-BEADS covalently linked to goat anti-mouse IgG IgG were purchased from the Pierce Chemical Company in Rockford, Illinois. Culture supernatants containing secreted anti-iNOS MAbs from clones 21C10-1D10, 2A1-F8, 1E8-B8, and 2D2-B2 were applied individually to aliquots of the suspended MAG-BEADS in order to load the beads with antibodies to iNOS. The particulate fraction containing iNOS was diluted 1:5 and applied to the pooled, wash, and resuspended anti-iNOS coated MAG-BEADS. The suspension was incubated overnight with gentle mixing to allow the particulate iNOS to bind to the antibodies coated to the MAG-BEADS before the tube containing the suspension was placed on the magnetic rack. All the beads congregated to the sides of the tube next to the magnets, and the iNOS-depleted solution (iNOS-depleted particulate fraction) was transferred and diluted to a final volume to yield a 1:10 dilution as compared to the stock particulate fraction. A 1:10 dilution of the stock iNOS particulate fraction was also prepared in sterile saline. Samples of the iNOS-depleted particulate fraction, of the stock particulate fraction, and of the iNOS loaded MAG-BEADS used to create the iNOS-depleted particulate traction, were all analyzed to determine if the particulate iNOS had been removed, Fig. 9. The iNOS bound to the anti-iNOS MAbs attached to the MAG-BEADS is determined in Fig. 10. These analyses showed that more than 90 percent of the particulate iNOS had been removed from the particulate fraction (iNOS-depleted particulate fraction) and that the iNOS was bound to the MAG-BEADS which had been loaded with the anti-iNOS MAbs.

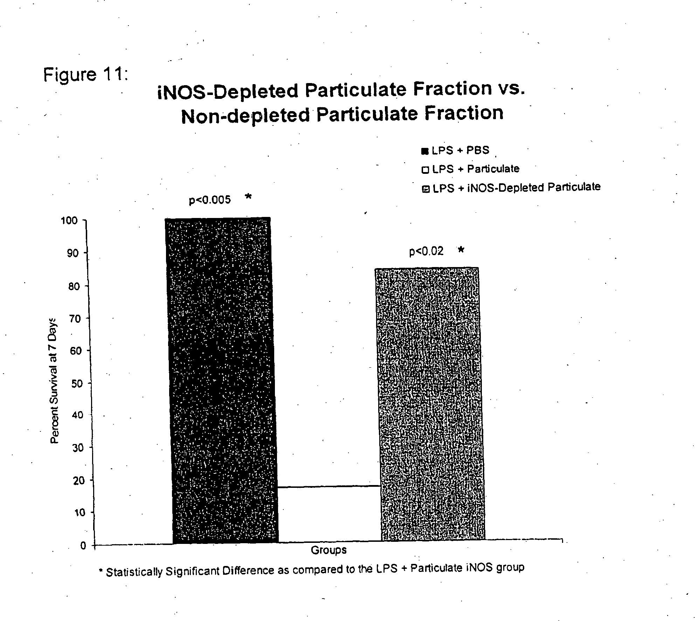

The effect that the iNOS-depleted particulate fraction had on the LPS-primed mice was compared to that seen with the stock (non-depleted) particulate fraction containing particulate iNOS. Groups of mice containing both genders were injected IP with a sub-lethal dose of LPS (2mg/kg body weight of LPS serotype 0111:B4 from *E. coli* obtained from the Sigma Chemical Company) in sterile saline. After four hours, all the mice primed with LPS became lethargic and developed diarrhea. The various groups of mice were then given a tail vein injection of either saline, stock particulate iNOS at a 1:10 dilution, or iNOS-depleted particulate fraction at a 1:10 dilution as compared to the starting stock suspension. Fig. 11 represents these definitive results. None of the mice that received a priming IP injection of LPS followed four hours later by a tail injection of saline showed any effect since they all survived seven days until the end of the experiment of this Example. However, only 17 percent (one out of six) of the mice that received a priming IP injection of LPS followed four hours later by a tail injection of particulate iNOS at a 1:10 dilution, survived for seven days. Significantly, 84 percent (five of six) of the LPS-primed mice that received a tail vein injection of the iNOS-deleted particulate fraction survived for seven days. When these data were compared, a high degree of statistically significant difference was found between the survival of the mice administered the particulate iNOS fraction and those administered saline (P<0.005 by Student's T-test) or the iNOS-depleted particulate fraction (P<0.02). There was no statistically significant difference between the LPS-primed mice that received a saline IV injection and those that received the iNOS-depleted particulate rraction. Thus, the specific removal of the particulate iNOS from the particulate fraction abolished the lethal effect seen in the LPS-primed mice that received the particulate iNOS fraction. It was concluded that (1) LPS priming was required for the'lethal effect of particulate iNOS to be exerted;. (2) particulate iNOS by itself or particulate iNOS in association with one or more proteins was responsible for the lethal effect observed in LPS-primed mice; and (3) removal of the particulate iNOS or particulate iNOS in association with one or more proteins, by absorption from solution using immobilized anti-iNOS MAbs, stopped the lethal effects asserted by the administration of the particulate iNOS.

### EXAMPLE III

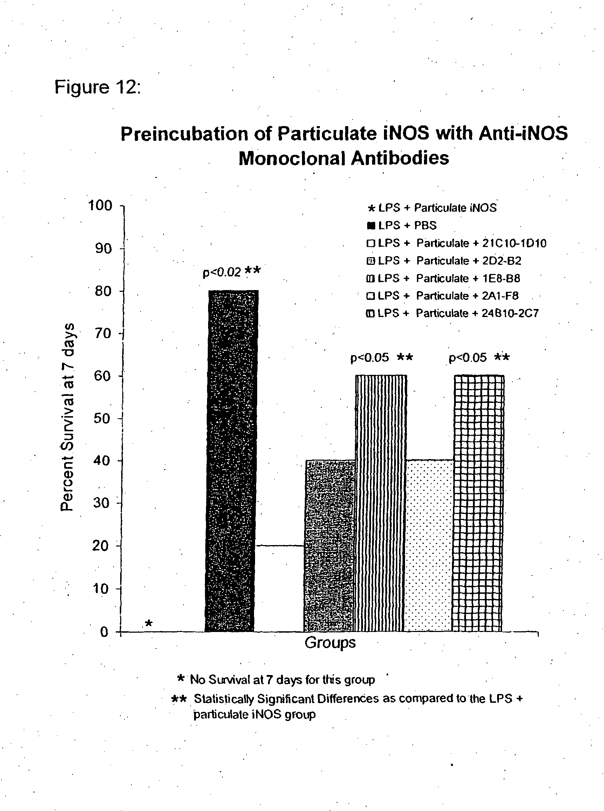

A second method was employed to study the ability of the anti-human iNOS MAbs of United States Patent 6,531,578 to inhibit the killing effect seen with particulate human iNOS in LPS-primed mice as a model for sepsis. Instead of physically removing the particulate iNOS from the particulate fraction as was performed in Example II, individual anti-iNOS MAbs contained,in ascites fluid were added directly to aliquots of the particulate fraction that contained particulate iNOS. The particulate iNOS fraction was allowed to bind to the anti-iNOS MAbs for 45 minutes before the material was injected IV into mice. Five different anti-iNOS MAbs were tested for their individual ability to inhibit (neutralize) the killing effect of the particulate human iNOS. Groups of mice were primed with a sub-lethal dose of LPS (2mg/kg body weight of LPS Serotype 0111:B4 from *E. coli* obtained from the Sigma Chemical Company) in sterile saline. After four hours all the LPS-primed mice became lethargic and developed diarrhea. The various groups of mice were given a tail vein injection of either saline, stock particulate iNOS at a 1:10 dilution, or stock particulate iNOS at a 1:10 dilution that had been preincubated for 45 minutes with one of five different anti-iNOS MAbs. Each of the five different anti-iNOS MAbs was used at a 1:50 dilution of the ascites fluid. The results varied and are shown in Fig. 12. All the LPS-primed mice that received a tail vein injection of the stock particulate iNOS diluted 1:10 in sterile saline died within the first 24 hours of the seven day experiment. In contrast, four out of five (80 percent) of the LPS-primed mice administered a saline tail vein injection survived seven days (P<0.02). The ability of the anti-iNOS MAbs to neutralize the lethal effect of the particulate iNOS varied depending on the MAb being tested. Of the five different anti-iNOS MAbs tested, anti-iNOS MAb 1E8-B8 and 24B10-2C10 were the best at neutralizing the lethal effects of the particulate iNOS on LPS-primed mice. In both cases, three out of five mice survived seven days (P<0.05). To other anti-iNOS MAbs (2D2-B2 and 2A1-F8) were also somewhat effective in stopping the mice from dying, i.e. two out of five mice in each of these groups survived seven days. One anti-iNOS MAb (21C10-1D10) was much less effective since only one out of five mice survived seven days. It was concluded that: (1) LPS priming is necessary for the particulate iNOS to be lethal; (2) that it is not necessary to remove physically the particulate iNOS from the solution in order to neutralize its lethality; (3) that anti-iNOS MAbs can neutralize the lethal effects of particulate iNOS on LPS-primed mice by binding to iNOS or by binding to the protein-protein complex containing particulate iNOS; and (4) that different anti-iNOS MAbs vary in their individual ability to neutralize the lethality of particulate iNOS either as particulate iNOS itself or in association with one or more proteins.

### EXAMPLE IV

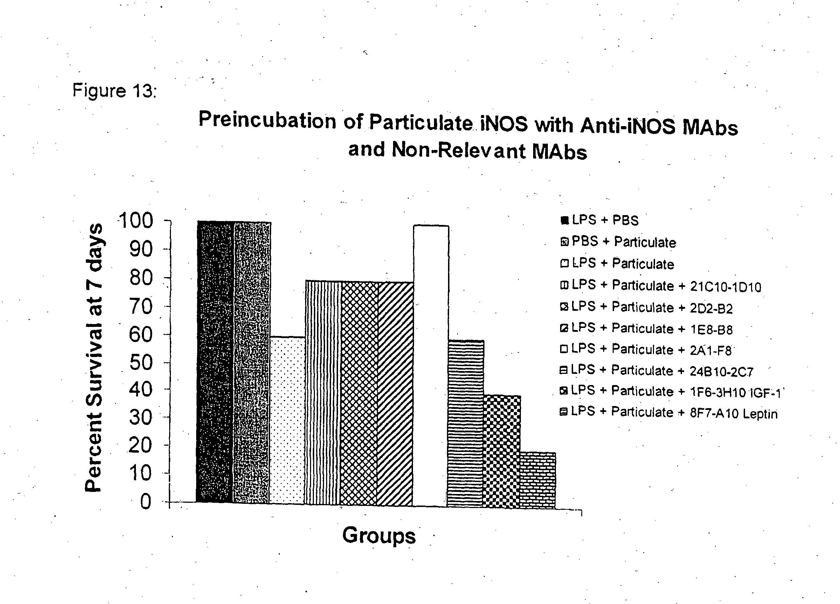

A lower priming dose of LPS and a lower dose of particulate iNOS were employed than that used in Examples I-III on groups of mice. Two ascites fluids containing non-relevant MAbs were also tested as controls. The non-relevant controls MAbs included one specific for insulin-like growth factor 1 (IGF-1: MAb clone 1F6-3H10) and one MAb specific for human leptin (MAb clone 8F7-A10). Groups of mice were primed with a lower sub-lethal dose of LPS (1 mg/kg body weight of LPS Serotype 0111:B4 from *E. coli.* obtained from the Sigma Chemical Company) in sterile saline. After four hours, all the LPS primed mice became lethargic and developed diarrhea. The various groups of mice were then give a tail vein injection of either saline, stock particulate iNOS at a 1:20 dilution, or stock particulate iNOS at a 1:20 dilution that had been preincubated for 45 minutes with one of five different anti-iNOS MAbs or one of two non-relevant MAbs, above-identified. The non-relevant MAbs and the anti-iNOS MAbs were each used at a dilution of 1:50 of the ascites fluid. The results were variable and are shown on Fig. 13. By using the lower amount of priming LPS and of particulate iNOS on mice, three out of five mice survived for seven days. As is shown in Fig. 13, anti-iNOS MAb clone 2A1-F8 was best at neutralizing the lethal effects of the particulate iNOS, since five out of five mice survived in this group until the end of the seven day experiment. However, when the particulate iNOS was preincubated with the ascites fluid containing either of the two non-relevant MAbs the survival rate at seven days was lower as compared to the iNOS particulate fraction. This suggests: (1) that one or more components in the ascites fluid increases the lethal effect of the particulate iNOS, or (2) that the individual mice in the group primed with LPS and then administered the particulate iNOS were less sensitive to the lethal effects of the particulate iNOS. Consequently, more members of the group survived than would have been expected to survive under similar conditions. If it is the latter case, then using more mice per group would resolve this statistical problem. If it is the former, where the ascites fluid somehow amplified the lethality of the particulate iNOS, then the ability of the anti-iNOS MAbs to neutralize the lethal effect is being underestimated. When the survival of the group of mice administered the particulate iNOS preincubated with anti-iNOS MAb 2A1-FA was compared to the survival of the mice in the two non-related MAb groups, a statistically significant difference was found - in other words, when compared to the group preincubated with an anti-IGF-1 MAb, P<0.05, and when compared to the group preincubated with anti-leptin MAb, P<0.02. It was concluded in this Example that: (1) lower doses of LPS and particulate iNOS increase the seven-day survival rate of the mice; (2) the mice had to be primed with LPS for the lethal effect of the particulate iNOS to be observed; (3) preincubation of the particulate iNOS with anti-iNOS MAbs increased the seven day survival rate of the mice; and (4) ascites fluid by itself was not responsible for the beneficial effects observed with the anti-iNOS MAbs.

### EXAMPLE V

In order to determine the binding activity of certain of the humanized anti-hiNOS MAbs the following tests were performed with respect to the relevant cognate peptide and to hiNOS protein.

The three humanized anti-hiNOS chimeric MAbs, developed by genetic engineering procedures hereinbefore described (humanized 1E8-B8, humanized 2D10-2H9 and humanized 24H9-1F3) were tested for their ability to recognize and to bind to the same epitopes to which the original mouse MAbs had been found to bind. Mouse anti-hiNOS MAb clones 1E8-B8 and 24H9-1F3 had previously been shown to bind to synthetic peptide analogs of specific regions of human iNOS in the teachings of United States Patent 6,531,578. Mouse anti-hiNOS MAb 1E8-B8 had been found to bind to peptide PS-5183 (peptide G11, which is hiNOS[985-1002]), and mouse anti-hiNOS MAb 24H9-1F3 has been found to bind to peptide PS-5166 (peptide F6, which is hiNOS[781-798]). The amino acid sequences of peptides G11 and F6 are disclosed in United States Patent 6,531,578. The genetically engineered chimeric mouse/human 1E8-B8 and 24H9-1F3 MAbs were first tested for binding to their respective epitope peptide analogs in parallel with the original mouse MAbs in ELISA titration experiments.

The original mouse 1E8-B8 MAb bound to the G11 peptide, hiNOS[985-1002], and the amount bound decreased with increasingly dilute MAb, as is expected for an antibody titration experiment. Similarly, the humanized 1E8-B8 bound to the immobilized G11 peptide. However, the goat anti-mouse IgG-HRP conjugated "detection" Ab did not bind to the humanized 1E8-B8 MAb which demonstrated that the antibody binding site was no longer contained on a mouse IgG molecule. However, the goat anti-human IgG-HRP conjugated "detection" Ab did bind to the chimeric 1E8-B8 MAb, which indicated that the binding site had been fused into a human IgG₁ molecule.

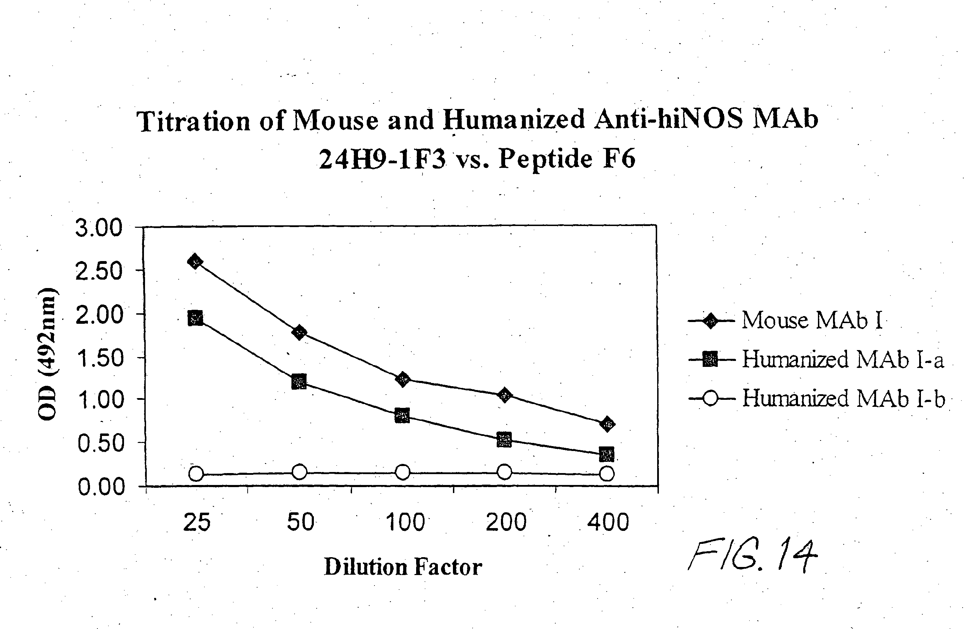

Similarly, when the original mouse 24H9-1F3 MAb and the chimeric mouse/human 24H9-1F3 MAb were tested for binding to the F6 peptide, hiNOS[781-798], the mouse MAb titered out with increasing dilution as expected, as did the humanized MAb, when goat anti-human IgG-HRP was used as the "detection" Ab. No color developed in this colormetric ELISA when goat anti-mouse IgG-HRP conjugate was used as the "detection" Ab with the humanized 24H9-1F3 MAb. This again demonstrated that a mouse binding site had been fused into a human IgG molecule, Fig. 14.

### EXAMPLE VI

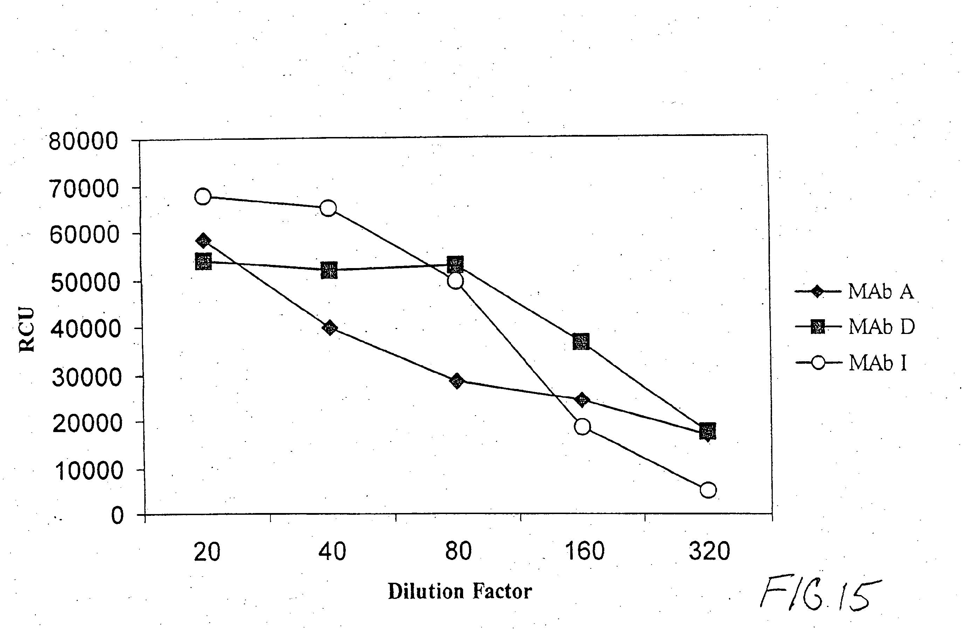

In addition to testing the humanized anti-hiNOS MAbs for binding to synthetic peptides, humanized 1E8-B8, humanized 2D10-2H9 and humanized 24H9-1F3 were amplified and expanded to obtain milligram quantities of the same. These humanized MAbs were tested for their ability to bind to hiNOS protein in a sandwich EIA format. In this format, a "catch" or "capture" Ab was immobilized on the surface of the wells of a microtiter plate, and was used to bind, and thereby immobilize, hiNOS added to the wells. Once bound to the "catch" or "capture" Ab, a second Ab was bound to a different site on the immobilized hiNOS protein on the surface of the well. In this specific case, the second antibody bound was one of the three humanized anti-hiNOS MAbs, humanized 1E8-B8, humanized 2D10-21H9, or humanized 24H9-1F3. As is shown in Figure 15, all three of the chimeric anti-hiNOS MAbs bound to the immobilized hiNOS protein in this sandwich EIA format. Further, these chimeric anti-hiNOS MAbs could only be detected as being bound to the immobilized hiNOS when a goat anti-human IgG-HRP conjugated "detection" third antibody was used. If a goat anti-mouse IgG-HRP conjugate was used as the "detection" third antibody in this chemiluminescent sandwich EIA, no chemiluminescence above background was measured. Thus, it was concluded: (1) that the humanized anti-hiNOS MAbs can bind to their respective epitopes on the hiNOS protein; (2) that the original mouse binding site has been fused into a human IgG molecule; and (3) that anti-mouse IgG-HRP conjugated antibody does not bind to these humanized anti-hiNOS MAbs.

### EXAMPLE VII

As previously described for the parent mouse anti-hiNOS MAbs, Example I, the ability of the chimeric mouse/human anti-hiNOS MAb 1E8-B8 to remove or deplete hiNOS from the particulate fraction obtained from cytokine induced and lysed DLD-1-5B2 cells was assessed. The mouse model of sepsis, Examples I and II, utilized a sub-lethal dose of LPS to prime the mice and then four hours later a suspension of particulate fraction of cytokine induced DLD-1-5B2 cells that had been lysed and fractionated by centrifugation was injected IV. Two different methods for cell lysis were used, two rapid freeze/thaw cycles (cell lysis method A) or hypotonic shock (cell lysis method B). Following cell lysis by either of these two methods the cell lysate was fractionated by centrifugation. In centrifugation method 1, the cell lysate was centrifuged at high speed (16,000 x g) to produce a high speed particulate fraction or pellet and a high speed supernatant fraction. In centrifugation method 2, the cell lysate was centrifuged at a low speed (300 x g) to produce a low speed particulate fraction or pellet. The low speed supernatant was then centrifuged at high speed (16,000 x g) to product a high speed particulate fraction and a high speed supernatant. Both procedures yielded a particulate fraction that contained the mouse killing activity in the mouse model of sepsis of Example I. By employing centrifugation method 2, the lethal activity was found exclusively in the low speed particulate fraction or pellet.

Using centrifugation method 1, the residual hiNOS-depleted high speed particulate fraction after treatment with Mag-beads loaded with humanized anti-hiNOS MAb 1E8-B8 was tested for its ability to kill LPS-primed mice in the animal model of sepsis of Example I. Two series of experiments were conducted both of which used Balb/c mice primed with a sub-lethal dose of LPS (2 mg/kg body weight) and high speed particulate fraction prepared by centrifugation method 1. In one series of experiments, the untreated high speed particulate fraction was used at a dilution that resulted in 80% of the mice dying (N=5, LD₈₀). When this same high speed particulate fraction was treated with the humanized anti-hiNOS MAb loaded Mag-beads and the residual hiNOS-depleted high speed particulate fraction tested at the same dilution as the untreated high speed particulate fraction, no mice died - all five mice survived for the 7 days of the experiment (P < 0.01 by Student's T-test). In the second series of experiments, the untreated particulate high speed fraction was used at a 50% higher concentration which resulted in all the mice dying (N=5, LD₁₀₀). When the residual hiNOS-depleted particulate fraction was tested at the same concentration, again no mice died - all five mice survived for the 7 days of the experiment (P < 0.001). Thus, the physical removal of the high speed particulate hiNOS, fragments of hiNOS in the high speed particulate fraction, vesicle associated with hiNOS and with hiNOS fragments, and the proteins associated with hiNOS and with fragments of hiNOS in the high speed particulate fraction bound to immobilized, humanized anti-hiNOS MAb 1E8-B8, resulted in a loss of the lethal effects and in a dramatic increase in survival.

### EXAMPLE VIII

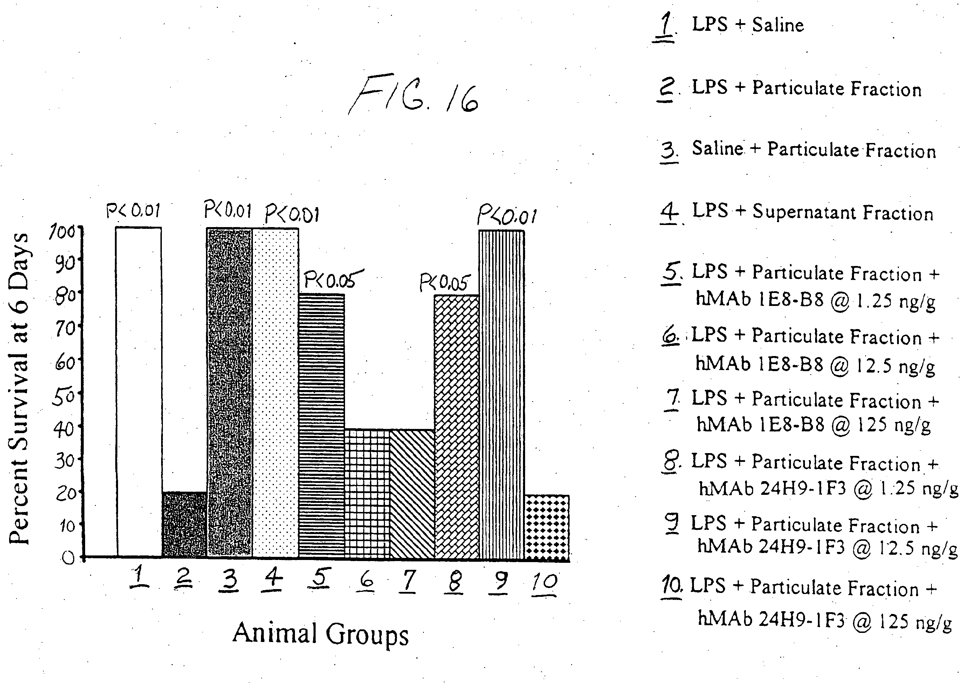

The ability of the humanized chimeric anti-hiNOS MAbs to neutralize the lethal activity contained in the particulate fraction of cytokine induced and lysed DLD-1-5B2 cells was assessed in a series of in vivo mouse experiments. These experiments were similar to the ones previous described for the parental mouse anti-hiNOS MAbs, Examples III and IV, except: (1) the mouse anti-hiNOS MAbs had been replaced by humanized anti-hiNOS MAbs 1E8-B8 and 24H9-1F3 and (2) the MAbs and particulate fraction were mixed immediately before IV injection and were not preincubated for 45 minutes before injection. The ability of these two humanized anti-hiNOS MAbs to neutralize, and thereby, to protect, LPS-primed mice from a lethal challenge of the hiNOS-containing particulate fraction (centrifugation method 1 of Example VII) obtained from cytokine induced and lysed DLD-1-5B2 cells was tested (Fig. 16). In these experiments, the neutralizing ability (protective effect) of these two humanized anti-hiNOS MAbs was tested at three different doses, 1.25 ng/g body weight, 12.5 ng/g body weight, and 125 ng/g body weight. The protective effect was found to be dose-dependent. At the lower doses used in these experiments, no statistically significant difference was observed between the MAb-treated groups and the untreated group, but a trend to protect was found (Fig. 16). However, at the highest concentration of humanized 1E8-B8 tested, a statistically significant difference between the MAb-treated group and untreated group was found (P < 0.05 by Student's T-test). Also, the protective effect of humanized anti-hiNOS MAb 24H9-1F3 was found to be statistically different at the two highest doses tested as compared to the untreated group of animals. When this MAb was used at 12.5 ng/g body weight, all 5 animals survived (P< 0.01), and when used at 125 ng/g body weight, 4 of the 5 animals survived (P < 0.05). These findings are similar to those made using the parental mouse anti-hiNOS MAbs prior to being genetically engineered into chimeric mouse/human IgG₁ molecules. These results further confirm the ability of these MAbs to neutralize the lethal activity contained in the particulate fraction of cytokine induced and lysed DLD-1-5B2 cells in a dose-dependent manner by binding to the particulate hiNOS, and thereby sterically hindering it from binding to susceptible cells for exertion of its lethal effect(s).

### EXAMPLE IX

Experiments were conducted to identify any proteins associated with hiNOS in the particulate fraction of cytokine induced DLD-1-5B2 cells. Anti-hiNOS MAbs were immobilized on Mag-beads to bind the particulate hiNOS and the hiNOS associated with other proteins in the particulate fraction. After treating the particulate fraction obtained from cytokine induced DLD-1-5B2 cells with immobilized anti-hiNOS MAb 1E8-B8 on Mag-beads, the Mag-beads were washed to remove unbound materials, and then the proteins were stripped off the immobilized antibody. The isolated proteins were separated by native gel PAGE; the protein bands stained with Coomassie brilliant blue; and the gel de-stained to visualize the protein bands. The protein bands that differed from those seen in gels from uninduced DLD-1-5B2 cells were sliced from the gel. The protein(s) contained in an individual band were digested with trypsin to produce tryptic fragments. The peptide fragments were isolated and subjected to LC/MS/MS for analysis in order to determine their individual amino acid sequences. The amino acid sequences determined by the LC/MS/MS analyses were compared to all known protein sequences contained in the GeneBank database. Proteins that have amino acid sequence homology with multiple tryptic peptide fragments and that have a statistically significant portion of their overall amino acid sequence covered by tryptic peptide fragments were identified and tabulated (Table 1). For example, as described in Table 1, PDI is human protein disulfide isomerase precursor (which is also known as Prolyl 4-hydroxylase beta subunit). The amino acid sequence of ten different tryptic peptides matched exactly with various regions of this protein's amino acid sequence and cover 23.0% of the overall sequence. The ladder scores for the individual peptide fragments range from 58.0 to 95.9, and the relative level of confidence in the prediction that this specific protein exists in this sample is greater than 80%. Thus, the mass spectral results indicate that this protein is contained in the band excised from the PAGE gel. The GeneBank accession number for this protein is gi|2507460. The same information for the thirteen other human proteins identified in association with hiNOS or fragments of hiNOS in the particulate fraction of cytokine induced DLD-1-5B2 cells is shown in Table 1.

**TABLE 1**

| | Protein | Number of Matches of Typtic Peptide Fragments | % Amino Acid Sequence Covered | Acession Number |
|---|---|---|---|---|
| 1 | PDI Protein disulfide isomerase precursor | 10 | 23.0 | gi\|2507460 |
| 2 | Non-Oncogenic Rho GTPase-Specific GTP exchange Factor | 12 | 22.2 | gi\|5199316 |
| 3 | Voltage-dependent Calcium channel alpha 1G subunit isoform 3 | 9 | 13.7 | gi\|38505292 |
| 4 | PDA4 Protein disulfide isomerase A4 precusor | 4 | 8.5 | gi\|119530 |
| 5 | Reticulocalbin 1 precusor | 4 | 14.8 | gi\|2493462 |
| 6 | Histone H2B | 4 | 27.0 | gi\|7387738 |
| 7 | Heat shock protein HSP 90-beta | 3 | 5.2 | gi\|17865718 |
| 8 | 78 kD Glucose regulated protein precursor | 8 | 16.8 | gi\|14916999 |
| 9 | Calreticulin | 9 | 22.5 | gi\|30583735 |
| 10 | Nuclear envelope pore membrane protein POM 121 | 6 | 7.8 | gi\|12643948 |
| 11 | PRIP-interacting protein PIPMT | 9 | 4.5 | gi\|14278850 |
| 12 | KIAA0833 | 15 | 12.2 | gi\|20521670 |
| 13 | Parathyroid hormone/parathyroid hormone-related peptide receptor | 4 | 9.7 | gi\|1172742 |
| 14 | Large-conductance calcium-activated potassium channel | 8 | 10.9 | gi\|537439 |

Each of the human proteins of Table 1 identified by tryptic digestion and LC/MS of the tryptic peptide fragments in bands excised from native PAGE gels of the particulate fraction, was obtained from induced and lysed DLD-1-5B2 cells that bound specifically to our anti-hiNOS MAbs. Therefore, each of these proteins was associated with hiNOS in the particulate fraction obtained from cytokine induced and lysed DLD-1-5B2 cells.

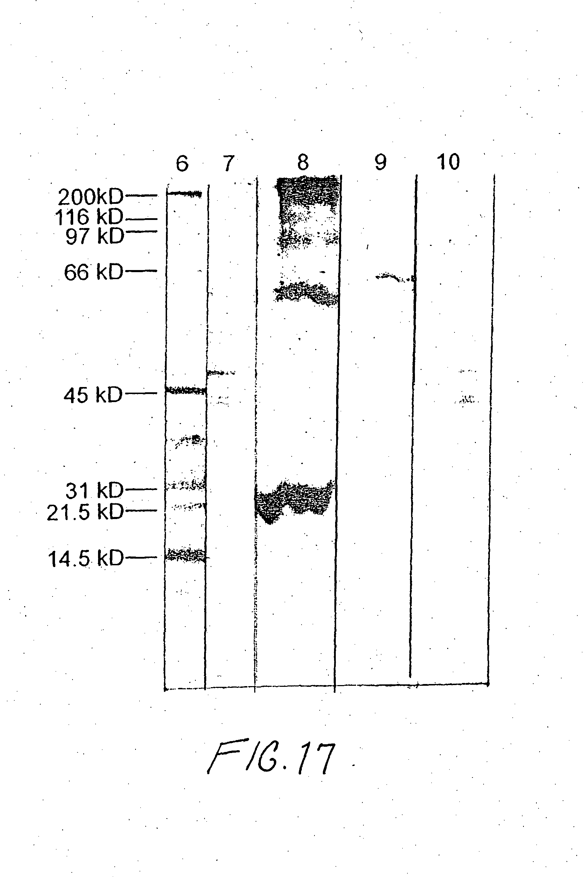

Antibodies for three of the proteins identified (Table 1) are commercially available, and were used to probe membranes for Western blots following SDS-PAGE separation of the proteins stripped off the anti-hiNOS MAb immobilized on Mag-beads, Fig. 17. These Western blots positively confirmed the presence of human PDI precursor, human HSP 90-beta, and histone H2B proteins in the material stripped off the anti-hiNOS immobilized Mag-beads. The removal of the proteins associated with hiNOS in the particulate fraction obtained for cytokine induced DLD-1-5B2 cells may also contribute to the decreased killing activity in the in vivo mouse model of sepsis that results from treatment with immobilized anti-hiNOS MAbs of Examples II, III, and VIII.

### EXAMPLE X

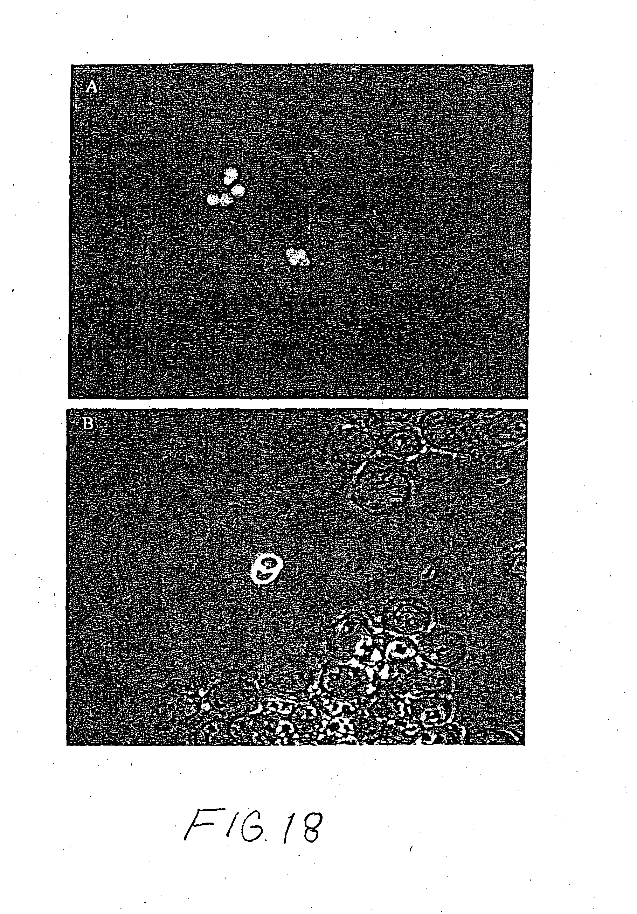

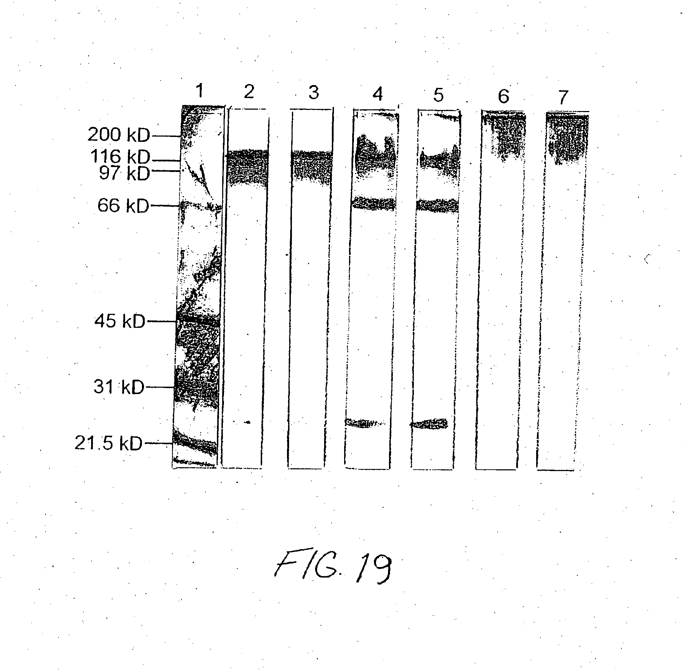

Immunofluorescent staining experiments were performed to determine if the hiNOS contained in the particulate fraction is located in the cellular membrane, or in vesicles, or in other structures, such as membrane fragments. Cytokine induced DLD-1-5B2 cells were lysed by hypotonic shock (cell lysis method B) of Example VII, in order to rupture the cellular membrane and release cellular components, the cellular components, and membrane structures, including blebbing vesicles, into the solution to avoid denaturing the proteins, as can occur with multiple freeze/thaw cycles. The solution containing the lysed cells was subjected first to low speed centrifugation (at 300 x g) to obtain a low speed particulate pellet. The low speed supernatant was then subjected to higher speed centrifugation (at 16,000 x g) to produce both a high speed particulate pellet and a high speed supernatant. When these three fractions were tested for their killing activity in LPS-primed mice, the lethal activity was only found in the low speed particulate fraction. Neither the high speed pellet nor the high speed supernatant was lethal when injected intravenously into LPS-primed mice. When the low speed particulate fraction was examined microscopically, two types of structures were observed. One was the cellular membrane of "ghost" cells, i.e. ruptured cell remnants, and the other was small vesicles which many times appeared linked together liked beads on a string (Fig. 18). When this preparation was stained by immunofluorescence using the anti-hiNOS MAbs of U.S. Patent 6,531,578 to immunolocalize the hiNOS, intense fluorescent staining was observed exclusively in the small vesicles. No IFA staining of hiNOS was observed in the "ghost" cells or in any other structure. The size of these vesicles (apoptoic bodies) and their intense IFA staining with the noted anti-hiNOS MAbs is very similar, if not identical, to that observed in the blood of human septic patients (Figs. 1-6). When these preparations were also analyzed by Western blot after SDS-PAGE separation of the proteins (Fig. 19), the high speed supernatant was found to contain intact hiNOS. The low speed particulate fraction contained a small quantity of intact hiNOS, but it also contained two hiNOS fragments that bound the anti-hiNOS MAb 2D2-B2 used in these experiments (Figure 19). It was repeatedly found that the high speed supernatant does not kill the LPS-primed mice in the animal model of sepsis of Example I while the low speed particulate fraction is lethal to the LPS-primed mice.

Further, in two series of experiments, (1) humanized MAb 1E8-B8 immobilized on Mag-beads, (2) peptide G-11 to which MAb 1E8-B8 binds, and (3) the particulate fraction obtained from cytokine induced DLD-1-5B2 cells that were lysed either by 2 freeze/thaw cycles (cell lysis method A of Example VII) or by hypotonic shock (cell lysis method B of Example VII), as described above, were used to recover the hiNOS and associated materials including vesicles bound to the anti-hiNOS loaded MAG-BEADS. After treating the two particulate fractions (one was prepared by cell lysis method A of Example VII and the other was prepared by cell lysis method B of Example VII) with humanized MAb 1E8-B8 immobilized on MAG-BEADS, the MAG-BEADS were washed to remove unbound material. The material bound to the anti-hiNOS loaded MAG-BEADS were competed-off the humanized anti-hiNOS MAb by incubating the loaded beads with a high concentration (100 µg) of peptide G-11. The material competed-off the humanized anti-hiNOS 1E8-B8 MAb bound to the MAG-BEADS was centrifuged, washed and fluorescently stained using other anti-hiNOS MAbs in the panel of United States Patent 6,531,578. In both series of experiments, vesicles were observed that fluoresced intensely, and no other material was observed to immunofluoresce. When these same two preparations were used to challenge mice primed with a sub-lethal dose of LPS in the mouse model of sepsis of Example I, both samples were found to possess the lethal activity initially discovered in the particulate fraction obtained from cytokine induced and lysed DLD-1-5B2 cells. Namely, with one of these recovered preparations, 2 out of 2 mice died, and with the other recovered preparation, 1 out of 2 mice died. When the proteins contained in the material competed-off the anti-hiNOS loaded MAG-BEADS were analyzed by Western blots using anti-hiNOS MAb clone 2D2-B2 that binds to the hiNOS[781-798], three main bands were found at apparent molecular weights of 131kD, 70 kD, and 27 kD for the material recovered from the particulate fraction prepared by cell lysis method A of Example VII. However, a single band at 70kD was found for the sample recovered from the low speed particulate fraction prepared by cell lysis method B of Example VII.

### EXAMPLE XI

In further experiments, of the type of Example VIII, displayed in Fig. 16, the lethal effect of the supernatant fraction obtained from cytoxine induced and lysed DLD-1-5B2 cells was tested in the *in vivo* mouse model of sepsis of Example I, and no killing activity was found. In a series of experiments, the ability of the supernatant fraction to protect mice primed with a sub-lethal dose of LPS was explored. In this series of experiments mice were primed with LPS as previously described, and were then challenged four hours later with one of three different treatments: (group 1, N=5) the particulate fraction from cytokine induced and lysed DLD-1-5B2 cells (method A of Example VII) at a dose that kills all the mice (LD₁₀₀); (group 2, N=5) the particulate fraction at an equivalent dose to group #1 plus a four times higher dose of the supernatant from cytokine induced and lyzed DLD-1-5B2 cells (method A of Example VII), simultaneously administered; and (group 3, N=5) a four times higher dose of the supernatant from cytokine induced and lyzed DLD-1-5B2 cells (method A of Example VII) administered 30 minutes prior to the administration of the particulate fraction. In these experiments, all the animals in groups 1 and 2 died. However, all the animals in group 3 survived (P < 0.001 by Student's T-test). Thus, the prior administration of the supernatant fraction was protective against a lethal challenge of the particulate fraction. The non-lethal supernatant fraction blocked the lethal cellular events that had resulted from the administration of the particulate fraction. Thus, the supernatant fraction blocked the binding of the lethal material to cells, and thereby protected the animal from a lethal dose of the particulate fraction.

## Claims

1. A therapeutic agent for use in the treatment of an illness selected from the group consisting of systemic inflammatory response syndrome, sepsis, severe sepsis, and septic shock,
comprising:
a monoclonal antibody recognizing human iNOS.

2. The agent for use of claim 1 in which said monoclonal antibody recognizing human iNOS comprises a monoclonal antibody neutralizing a cellular effect caused by a form of hiNOS, and the form of hiNOS is selected from the group consisting essentially of:
particulate hiNOS, fragments of particulate hiNOS, vesicle associated hiNOS, vesicle associated fragments of particulate hiNOS, particulate hiNOS associated with another protein, and fragments of particulate hiNOS associated with another protein.

3. The agent for use of claim 2 in which said monoclonal antibody is selected from the group consisting essentially of mouse and-hiNOS monoclonal antibody, mouse-human chimeric anti-hiNOS monoclonal antibody, humanized anti-hiNOS monoclonal antibody, and human anti-hiNOS monoclonal antibody.

4. A therapeutic device for use in the treatment of an illness in a mammalian subject generating hiNOS in its blood, in which the illness is selected from the group consisting of systemic inflammatory response syndrome, sepsis, severe sepsis, and septic shock,
comprising:
a device coated with a monoclonal antibody recognizing human iNOS.

5. The device for use of claim 4 in which said monoclonal antibody recognizing human iNOS comprises a monoclonal antibody neutralizing a cellular effect caused by a form of hiNOS, and the form of hiNOS is selected from the group consisting essentially of:
particulate hiNOS, fragments of particulate hiNOS, vesicle associated hiNOS, vesicle associated fragments of particulate hiNOS, particulate hiNOS associated with another protein, and fragments of particulate hiNOS associated with another protein.

6. The device for use of claim 4 in which said monoclonal antibody is selected from the group consisting essentially of mouse anti-hiNOS monoclonal antibody, mouse-human chimeric anti-hiNOS monoclonal antibody, humanized anti-hiNOS monoclonal antibody, and human anti-hiNOS monoclonal antibody.

## Patentansprüche

1. Ein therapeutisches Mittel zur Verwendung in der Behandlung einer Krankheit, gewählt aus der Gruppe bestehend aus systemischem inflammatorischem Response-Syndrom, Sepsis, schwerer Sepsis und septischem Schock,
das Folgendes umfasst:
einen monoklonalen Antikörper, der Human-iNOS erkennt.

2. Das Mittel zur Verwendung gemäß Anspruch 1, worin der monoklonale Antikörper, der Human-iNOS erkennt, einen monoklonalen Antikörper umfasst, der einen zellulären Effekt neutralisiert, der durch eine Form von hiNOS verursacht wird, und die Form von hiNOS gewählt ist aus der Gruppe, die im Wesentlichen aus Folgendem besteht:
partikuläre hiNOS, Fragmenten von partikulärer hiNOS, Vesikelassoziierte hiNOS, Vesikel-assoziierten Fragmenten von partikulärer hiNOS, partikulärer hiNOS assoziiert mit einem anderen Protein, und Fragmenten von partikulärer hiNOS, assoziiert mit einem anderen Protein.

3. Das Mittel zur Verwendung gemäß Anspruch 2, worin der monoklonale Antikörper gewählt ist aus der Gruppe, die im Wesentlichen aus Folgendem besteht: Maus-Anti-hiNOS-monoklonalem Antikörper, MauB-Human-chimärischem Anti-hiNOS-monoklonalem Antikörper, humanisiertem Anti-hiNOS-monoklonalem Antikörper und humanem Anti-hiNOS-monoklonalem Antikörper.

4. Eine therapeutische Vorrichtung zur Verwendung in der Behandlung einer Krankheit bei einem Säugetier-Individuum, die hiNOS in seinem Blut erzeugt, worin die Krankheit gewählt ist aus der Gruppe bestehend aus systemischem inflammatorischem Response-Syndrom, sepsis, schwerer Sepsis und septischem Schock, Folgendes umfassend:
eine Vorrichtung, beschichtet mit einem monoklonalen Antikörper, der Human-iNOS erkennt.

5. Die Vorrichtung zur Verwendung gemäß Anspruch 4, worin der monoklonale Antikörper, der Human-iNOS erkennt, einen monoklonalen Antikörper umfasst, der einen zellulären Effekt neutralisiert, der durch eine Form von hiNOS verursacht wird, und die Form von hiNOS gewählt ist aus der Gruppe, die im Wesentlichen aus Folgendem besteht:
partikulärer hiNOS, Fragmenten von partikulärer hiNOS, Vesikelassoziierter hiNOS, Vesikel-assoziierten Fragmenten von partikulärer hiNOS, partikulärer hiNOS assoziiert mit einem anderen Protein, und Fragmenten von partikulärer hiNOS, assoziiert mit einem anderen Protein.

6. Die Vorrichtung zur Verwendung gemäß Anspruch 4, worin der monoklonale Antikörper gewählt ist aus der Gruppe, die im Wesentlichen aus Folgendem besteht: Maus-Anti-hiNOS-monoklonalem Antikörper, Maus-Human-chimärischem Anti-hiNOS-monoklonalem Antikörper, humanisiertem Anti-hiNOS-monoklonalem Antikörper und humanem Anti-hiNOS-monoklonalem Antikörper.

## Revendications

1. Agent thérapeutique pour utilisation dans le traitement d'une maladie choisie dans le groupe constitué d'un syndrome de réponse inflammatoire systémique, une sepsie, une sepsie sévère et un choc septique,
comprenant :
un anticorps monoclonal reconnaissant iNOS humain.

2. Agent thérapeutique pour utilisation de la revendication 1 dans lequel ledit anticorps monoclonal reconnaissant iNOS humain comprend un anticorps monoclonal neutralisant un effet cellulaire causé par une forme de hiNOS, et la forme de hiNOS est choisie dans le groupe constitué essentiellement de :
hiNOS particulaire, des fragments de hiNOS particulaire, hiNOS associé à une vésicule, des fragments de hiNOS particulaire associé à une vésicule, hiNOS particulaire associé à une autre protéine, et des fragments de hiNOS particulaire associé à une autre protéine,

3. Agent thérapeutique pour utilisation de la revendication 2 dans lequel ledit anticorps monoclonal est choisi dans le groupe constitué essentiellement d'un anticorps monoclonal anti-hiNOS de souris, un anticorps monoclonal anti-hiNOS chimérique souris-humain, un anticorps monoclonal anti-hiNOS humanisé, et un anticorps monoclonal anti-hiNOS humain.

4. Dispositif thérapeutique pour utilisation dans le traitement d'une maladie chez un sujet mammifère générant hiNOS dans son sang, la maladie étant choisie dans le groupe constitué d'un syndrome de réponse inflammatoire systémique, une sepsie, une sepsie sévère et un choc septique,
comprenant :
un dispositif revêtu d'un anticorps monoclonal reconnaissant iNOS humain.

5. Dispositif pour utilisation de la revendication 4, ledit anticorps monoclonal reconnaissant iNOS humain comprend un anticorps monoclonal neutralisant un effet cellulaire causé par une forme de hiNOS, et la forme de hiNOS est choisie dans le groupe constitué essentiellement de :
hiNOS particulaire, des fragments de hiNOS particulaire, hiNOS associé à une vésicule, des fragments de hiNOS particulaire associé à une vésicule, hiNOS particulaire associé à une autre protéine, et des fragments de hiNOS particulaire associé à une autre protéine.

6. Dispositif pour utilisation de la revendication 4, dans lequel ledit anticorps monoclonal est choisi dans le groupe constitué essentiellement d'un anticorps monoclonal anti-hiNOS de souris, un anticorps monoclonal anti-hiNOS chimérique souris-humain, un anticorps monoclonal anti-hiNOS humanisé, et un anticorps monoclonal anti-hiNOS humain.
